(19) Europäisches Patentamt
European Patent Office
Office européen des brevets

(11) **EP 3 426 154 B1**

(12) **EUROPEAN PATENT SPECIFICATION**

(45) Date of publication and mention
of the grant of the patent:
**11.03.2020 Bulletin 2020/11**

(21) Application number: **17714924.2**

(22) Date of filing: **10.01.2017**

(51) Int Cl.:
*A61B 6/00* *(2006.01)*          *A61M 5/145* *(2006.01)*
*A61M 5/168* *(2006.01)*          *A61M 5/00* *(2006.01)*
*A61M 5/142* *(2006.01)*          *G16H 20/17* *(2018.01)*
*G16H 40/63* *(2018.01)*

(86) International application number:
**PCT/US2017/012801**

(87) International publication number:
**WO 2017/155603 (14.09.2017 Gazette 2017/37)**

(54) **SYSTEM AND METHODS FOR PRE-INJECTION PRESSURE PREDICTION IN INJECTION PROCEDURES**

SYSTEM UND VERFAHREN ZUR VORHERSAGE DES VOREINSPRITZDRUCKS BEI INJEKTIONSVORGÄNGEN

SYSTÈME ET PROCÉDÉS DE PRÉVISION DE LA PRESSION DE PRÉ-INJECTION DANS DES PROTOCOLES D'INJECTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.03.2016 US 201615066130**

(43) Date of publication of application:
**16.01.2019 Bulletin 2019/03**

(60) Divisional application:
**19215920.0**

(73) Proprietor: **Bayer Healthcare LLC**
**Whippany, NJ 07981 (US)**

(72) Inventor: **KEMPER, Corey**
**Pittsburgh, PA 15206 (US)**

(74) Representative: **BIP Patents**
**c/o Bayer Intellectual Property GmbH**
**Alfred-Nobel-Straße 10**
**40789 Monheim am Rhein (DE)**

(56) References cited:
**EP-A1- 2 985 047          EP-A1- 2 990 073**
**WO-A1-2013/043868          US-A1- 2005 165 364**
**US-A1- 2006 079 765**

**Description**

**BACKGROUND**

The following information is provided to assist the reader in understanding technologies disclosed below and the environment in which such technologies may typically be used. The terms used herein are not intended to be limited to any particular narrow interpretation unless clearly stated otherwise in this document. References set forth herein may facilitate understanding of the technologies or the background thereof. The disclosure of all references cited herein are incorporated by reference.

In diagnostic injection procedures such as Computed Tomography (CT) Angiography, contrast (for example, a contrast medium including iodine) delivery rate and appropriate scan timing are very important to obtaining a high quality image. Often, relatively high flow rates are used in diagnostic studies (for example, in the range of 5 to 8 ml/s). A programmed flow rate in a diagnostic study may not, however, be achievable without exceeding a predetermined or programmed pressure limit (for example, 325 psi or 2241 kPa).

In a number of injectors, if a measured pressure exceeds the programmed pressure limit or pressure threshold value during an injection, pressure control algorithms of the injectors dynamically reduce the flow rate to ensure that disposables (that is, disposable fluid path elements or flow path elements such as syringes, tubing, catheter, etc.) and the patient's injection site are not damaged. The disposables form a fluid path or flow path which defines the path via which the fluid travels from a container (for example, a syringe) placed in operative connection with the injector apparatus to the patient. The terms "fluid path" and "flow path" are used herein interchangeably. The pressure limit may, for example, be set by the manufacturer or the user. Unfortunately, flow reduction resulting from reaching a pressure limit results in a change in the length of the contrast bolus, which means that the scan timing should be also adjusted. However, because the flow rate reduction occurs mid-injection, the flow reduction solves only the problem of not exceeding the pressure limit. There is no way for the clinician to readjust the injection duration, scan duration, or scan delay that were determined based upon the original flow rate. The clinician is simply left with a suboptimal or even unusable diagnostic image.

Document EP 2 990 073 A1 discloses a system for enabling delivery of fluid in an injection procedure.

Document WO 2013/043868 discloses a fluid pump device which is operable by a drive and actuating system. Document US 2006/079765 A1 discloses a contrast media injection system.

It is, therefore, desirable, to predict in advance of a diagnostic injection if the planned injection protocol will give rise to a pressure that exceeds the predetermined programmed pressure limit.

**SUMMARY**

A fluid injection apparatus includes at least one pressurizing system, at least a first fluid path operably connectible to the at least one pressurizing system to transport fluid pressurized by the pressurizing system. The at least a first fluid path includes a tubing set and a catheter. The fluid injection apparatus also includes a control system operably associated with the at least one pressurizing system and including an input system, a user interface system, a processor system, and a memory system. The input system provides for input of an initial injection protocol of an injection procedure according to which at least one fluid is intended to be injected into a patient. The fluid injection apparatus further includes at least one model stored in the memory system and executable by the processor system to predict, before carrying out the initial injection protocol, if a pressure threshold value would be reached in the at least a first fluid path if the initial injection protocol were carried out; the at least one model determining if the pressure threshold value would be reached on the basis of a flow rate of the at least one fluid, at least one catheter characteristic, and a viscosity of the at least one fluid. The at least one model is determined experimentally for a plurality of fluids of different viscosities, for a plurality of catheters of different catheter characteristics, and for at least one tubing set of like kind to the tubing set of the first fluid path. Upon determining via the at least one model that the pressure threshold value would not be reached, the processor system enables the initial injection protocol to be carried out by the fluid injection apparatus. Upon determining via the at least one model that the pressure threshold value will be reached, the processor system either (I) alerts an operator via the user interface system, whereupon the operator is enabled to choose between (a) permitting the initial injection protocol to be carried out by the fluid injection apparatus as programmed and (b) effecting a change to update the initial injection protocol for input into the at least one model or (II) automatically effects a change to update the initial injection protocol to create an updated injection protocol so that the expected pressure level will be determined via the at least one model to be less than or equal to the pressure threshold value, thereby enabling the updated injection protocol to be used to introduce the at least one fluid into the fluid path. The pressure threshold value may, for example, be set by one of (i) the operator of the fluid injection apparatus and (ii) a manufacturer of the fluid injection apparatus.

In a number of embodiments, effecting a change to update the initial injection protocol for input into the at least one model includes at least one of (a) changing the flow rate of the fluid, (b) changing the at least one catheter characteristic, and (c) changing the viscosity of the at least one fluid. The at least one model may, for example, be adapted to receive data from at least one previous injection procedure with the fluid injection apparatus to update the at least one model on the basis of the data from the at least one previous injection procedure with the fluid injection apparatus. The at least one model may, for example, be adapted to receive data from at least one previous injection procedure with another fluid injection apparatus to update the at least one model on the basis of the data from the at least one previous injection procedure with the another fluid injection apparatus.

In a number of embodiments, if it is determined via the at least one model that the pressure threshold value would be reached if the initial injection protocol were carried out, effecting of a change to update the initial injection protocol as chosen by the operator includes at least one of (i) a flow rate of the at least one fluid, (ii) a concentration of the at least one fluid, (iii) a ratio of one fluid of the at least one fluid to another fluid of the at least one fluid and (iv) a temperature of the at least one fluid. The at least one model may, for example, be determined experimentally for a plurality of fluids of different viscosities and for a plurality of catheters of different gauges used in connection with a plurality of tubing sets, wherein at least one of the plurality of tubing sets is of a like kind to the tubing set.

A method of predicting peak pressure within a fluid path, which includes a tubing set and a catheter into which at least one fluid is introduced under pressure by a pressurizing system of a fluid injection apparatus, includes (a) inputting an initial injection protocol into the fluid injection apparatus according to which the at least one fluid is intended to be introduced into the fluid path; (b) predicting an expected peak pressure level that would result in the fluid path if the initial injection protocol were to be used as intended to introduce the at least one fluid into the fluid path. The expected peak pressure level is determined before commencement of the initial injection protocol according to at least one model on the basis of a flow rate of the at least one fluid, at least one catheter characteristic, and a viscosity of the at least one fluid. The at least one model is determined experimentally for a plurality of fluids of different viscosities, for a plurality of catheters of different catheter characteristics, and for at least one tubing set of like kind to the tubing set of the fluid path. The pressure threshold value may, for example, be set by one of (i) the operator of the fluid injection apparatus and (ii) a manufacturer of the fluid injection apparatus.

In a number of embodiments, the method further include (c) if the expected pressure level is less than or equal to a pressure threshold value, enabling the initial injection protocol to be used as intended to introduce the at least one fluid into the fluid path; and (d) if the expected pressure level exceeds the pressure threshold value, performing one of: (I) alerting an operator of the method thereof whereupon the operator is enabled to choose between one of (i) permitting the initial injection protocol to be used as intended to introduce the at least one fluid into the fluid path and (ii) effecting a change to update the initial injection protocol for input into the at least one model; and (II) automatically effecting a change to update the initial injection protocol to create an updated injection protocol so that the expected pressure level will be determined via the at least one model to be less than or equal to the pressure threshold value thereby enabling the updated injection protocol to be used to introduce the at least one fluid into the fluid path.

Effecting a change to update the initial injection protocol for input into the at least one model may, for example, include changing the flow rate of the at least one fluid, changing the at least one catheter characteristic, or changing the viscosity of the at least one fluid.

In a number of embodiments, the method further includes inputting data from at least one previous injection procedure with the fluid injection apparatus into the at least one model and updating the at least one model on the basis of the data from the at least one previous injection procedure with the fluid injection apparatus. In a number of embodiments, the method further includes inputting data from at least one previous injection procedure with another fluid injection apparatus into the at least one model and updating the at least one model on the basis of the data from the at least one previous injection procedure with the another fluid injection apparatus.

In a number of embodiments, if it is determined via the at least one model that the pressure threshold value would be reached if the initial injection protocol were carried out, effecting of a change to update the initial injection protocol as chosen by the operator includes at least one of (i) a flow rate of the at least one fluid, (ii) a concentration of the at least one fluid, a (iii) a ratio of one fluid of the at least one fluid to another fluid of the at least one fluid and (iv) a temperature of the at least one fluid.

The at least one model may, for example, be determined experimentally for a plurality of fluids of different viscosities and for a plurality of catheters of different gauges used in connection with a plurality of tubing sets, wherein at least one of the plurality of tubing sets is of a like kind to the tubing set.

A non-transitory computer readable storage medium has instructions stored thereon, that when executed by a processor, perform actions including: storing input of at least a portion of an initial injection protocol into a fluid injection apparatus according to which at least one fluid is intended to be introduced into a fluid path; and predicting an expected peak pressure level that would result in the fluid path if the initial injection protocol were to be used as intended to introduce the at least one fluid into the fluid path. The expected peak pressure level is determined before commencement of the initial injection protocol according to at least one model on the basis of a flow rate of the at least one fluid, at least one catheter characteristic, and a viscosity of the at least one fluid. The at least one model is determined experimentally for a plurality of fluids of different viscosities, for a plurality of catheters of different catheter characteristics, and for at least one tubing set of like kind to a tubing set of the fluid path.

The non-transitory computer readable storage medium may further have instructions stored thereon, that when executed by a processor, perform actions including: enabling the initial injection protocol to be carried out by the fluid injection apparatus if the expected pressure level is less than or equal to a pressure threshold value; and if the expected pressure level exceeds the pressure threshold value, performing one of: (I) alerting an operator of the method thereof whereupon the operator is enabled to choose between one of (i) permitting the initial injection protocol to be used as intended to introduce the at least one fluid into the fluid path and (ii) effecting a change to update the initial injection protocol for input into the at least one model; and (II) automatically effecting a change to update the initial injection protocol to create an updated injection protocol so that the expected pressure level will be determined via the at least one model to be less than or equal to the pressure threshold value thereby enabling the updated injection protocol to be used to introduce the at least one fluid into the fluid path.

The present devices, systems, and methods, along with the attributes and attendant advantages thereof, will best be appreciated and understood in view of the following detailed description taken in conjunction with the accompanying drawings.

## BRIEF DESCRIPTION OF THE DRAWINGS

Figure 1 illustrates data from studies of maximum achievable flow rates in a fluid injection apparatus for contrast media of differing viscosities using catheters of various diameters.

Figure 2A illustrates maximum achievable flow rate by viscosity for catheters of various diameters.

Figure 2B illustrates a histogram of the error in prediction of pressure in studies of a fluid injection apparatus of the present invention.

Figure 3 illustrates schematically a fluid injection apparatus or system according to an embodiment of the present invention.

Figure 4 illustrates studies of the temperature dependence of the viscosity of several contrast media.

Figure 5 illustrates a flowchart of a method according to an embodiment of the present invention.

## DETAILED DESCRIPTION

It will be readily understood that the components of the embodiments, as generally described and illustrated in the figures herein, may be arranged and designed in a wide variety of different configurations in addition to the described representative embodiments. Thus, the following more detailed description of the representative embodiments, as illustrated in the figures, is not intended to limit the scope of the embodiments, as claimed, but is merely illustrative of representative embodiments.

Reference throughout this specification to "one embodiment" or "an embodiment" (or the like) means that a particular feature, structure, or characteristic described in connection with the embodiment is included in at least one embodiment. Thus, the appearance of the phrases "in one embodiment" or "in an embodiment" or the like in various places throughout this specification are not necessarily all referring to the same embodiment.

Furthermore, described features, structures, or characteristics may be combined in any suitable manner in one or more embodiments. In the following description, numerous specific details are provided to give a thorough understanding of the embodiments. One skilled in the relevant art will recognize, however, that the various embodiments can be practiced without one or more of the specific details, or with other methods, components, materials, et cetera. In other instances, well known structures, materials, or operations are not shown or described in detail to avoid obfuscation.

As used herein and in the appended claims, the singular forms "a," "an", and "the" include plural references unless the context clearly dictates otherwise. Thus, for example, reference to "a processor" includes a plurality of such processors and equivalents thereof known to those skilled in the art, and so forth, and reference to "the processor" is a reference to one or more such processors and equivalents thereof known to those skilled in the art, and so forth. Recitation of ranges of values herein are merely intended to serve as a shorthand method of referring individually to each separate value falling within the range. Unless otherwise indicated herein, each separate value, as well as intermediate ranges of values, are incorporated into the specification as if individually recited herein. All methods described herein can be performed in any suitable order unless otherwise indicated herein or otherwise clearly contraindicated by the text.

The term "control system" or "controller," as used herein includes, but is not limited to, any circuit or device that coordinates and controls the operation of one or more input or output devices. For example, a controller can include a device having one or more processors, microprocessors, or central processing units (CPUs) capable of being programmed to perform input or output functions.

As used herein, the term "circuit" or "circuitry" includes. but is not limited to, hardware, firmware, software or combinations of each to perform a function(s) or an action(s). For example, based on a desired feature or need. a circuit may include a software controlled microprocessor, discrete logic such as an application specific integrated circuit (ASIC), or other programmed logic device. A circuit may also be fully embodied as software.

The term "processor system" or "processor," as used herein refers generally to logic circuitry hardware that responds to and processes instructions and includes, but is not limited to, one or more of virtually any number of processor systems or stand-alone processors, such as microprocessors, microcontrollers, central processing units (CPUs), and digital signal processors (DSPs), in any combination. A processor may be associated with various other circuits that support operation of the processor, such as a memory system (for example, random access memory (RAM), read-only memory (ROM), programmable read-only memory (PROM), erasable programmable read only memory (EPROM)), clocks, decoders, memory controllers, or interrupt controllers, etc. These support circuits may be internal or external to the processor or its associated electronic packaging. The support circuits are in operative communication with the processor. The support circuits are not necessarily shown separate from the processor in block diagrams or other drawings.

There are a number of factors that determine if a programmed injection protocol is achievable without reaching a predetermined pressure limit. These factors include the contrast viscosity (determined by contrast type, concentration and temperature), as well as the characteristics of disposable flow path components. Viscosities for a number of commercially available contrast agents at room temperature are set forth in Table 1.

**Table 1**

| Contrast Agent (concentration) (mg. I/mL) | Viscosity (cP) |
|---|---|
| ULTRAVIST® 300 | 8 |
| VISIPAQUE® 320 | 20 |
| OMNIPAQUE® 350 | 16 |
| ULTRAVIST® 370 | 18 |
| IOMERON® 400 | 25 |

ULTRA VIST (isopromide) is a contrast enhancing agent available from Bayer Aktiengesellschaft of Berlin, Germany. VISIPAQUE (iodixanol) is a contrast enhancing agent available from GE Healthcare of Buckinghamshire, UK. OMNIPAQUE (iohexol) is a contrast enhancing agent available from GE Healthcare. IOMERON (iomerprol) is a contrast enhancing agent available from Bracco S.p.A. of Milan, Italy.

From Table 1, it is seen that there is generally an upward trend in viscosity with increasing concentration. However, it is not a strictly direct relationship. The viscosity also depends on the contrast enhancing molecule. As seen in Table 1, VISIPAQUE 320 (320 mg I/ml) actually has a higher viscosity than ULTRA VIST 370 (370 mg I/ml). Also, viscosity generally decreases with increasing temperature. For example, a contrast agent warmed to body temperature (recommended by many manufacturers to lower the viscosity and increase patient comfort) will have a viscosity about 1/2 the viscosity of the same contrast agent at room temperature.

As described above, other contributing factors include characteristics of the tubing and the patient catheter/cannula (such flow path components are sometimes referred to collectively as disposables as they are typically discarded after a single use with one patient). As used herein, the term "catheter" refers to an intravenous conduit used to introduce fluids into a patient's vascular or circulatory system and includes, for example, collectively, catheters and cannulas. The tubing is supplied with the injector and, unless a site is specifically using other flow path components (for example, extension sets), is typically consistent across diagnostic studies. The patient catheter, however, is variable. Depending on departmental policy, where the catheter is placed, the quality of the patient's veins, and the type of study to be performed, the catheter may vary in size/gauge from relatively small (for example, 24 gauge) to quite large (for example, 18 gauge). There is a balance in choosing the size of a catheter. A smaller catheter is easier to place and more comfortable for the patient, but a larger catheter is recommended for CT Angiography, to obtain sufficient iodine delivery rate (and therefore flow rate) to achieve a good image.

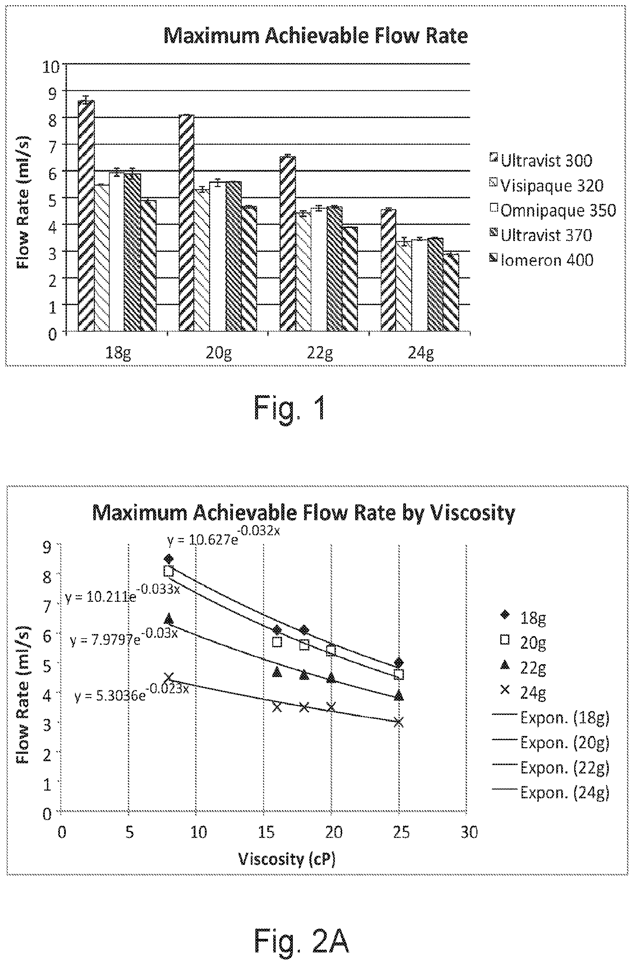

Figure 1 illustrates the results of a series of experimental studies to determine the achievable flow rate (given the predetermined pressure limit of the injector system setup) across a range of catheter sizes (18 gauge, 20 gauge, 22 gauge and 24 gauge) and several different contrast agents (ULTRAVIST 300, VISIPAQUE 320, OMNIPAQUE 350, ULTRIVIST 370, and IOMERON 400). In those studies, a particular flow set was used with catheters of the gauge indicated in the horizontal axis of the graph of Figure 1. In the studies, a MEDRAD® Stellant injector (a dual-syringe injector) was used and the flow set included the corresponding MEDRAD® low-pressure connector tubing (Part No. LPCT 160, a 60 inch length of low-pressure polymeric tubing). Both the MEDRAD® Stellant injector and the MEDRAD® low-pressure connector tubing are available from the Radiology business of the Pharmaceuticals Division of Bayer, which is located in Indianola, PA. In each study, the flow rate was increased until the predetermined pressure limit was reached. Each of the maximum achievable flow rates indicated in the graph of Figure 1 is 0.1 ml/s below the flow rate at which the pressure limit would have been reached. In the injector used, the flow rate could be changed in increments of 0.1 ml/sec. Recorded data from the experiments also provide, for example, a peak pressure achieved as a function of the flow rate and the tubing set characterization (including, catheter gauge), which can be provided in the model to enable prediction of peak pressure for the planned injection protocol.

As expected, lower viscosity contrast agents and larger catheters provided higher achievable flow rates. In Figure 2A, the maximum achievable flow rate results of Figure 1 are displayed as a function of the viscosity of the contrast agent for an injector apparatus with a pressure limit of 325 pounds per square inch (psi). There is a reasonably good negative exponential fit (as illustrated by the solid lines in Figure 2A) for the data for all four catheter types. Therefore, contrast agents with viscosity between those that are measured can yield a predicted maximum achievable flow rate.

Using data from studies as described herein, models were developed in which data such as catheter gauge and contrast type (viscosity) are to be entered as inputs into the models via a user interface. In that regard, catheter type/gauge and contrast viscosity are sufficient information for input into models hereof to, for example, predict a maximum achievable flow rate. The predicted maximum achievable flow rate may be compared to the maximum flow rate in a programmed injection protocol to determine if a pressure generated in executing the injection protocol will approach or exceed a pressure limit.

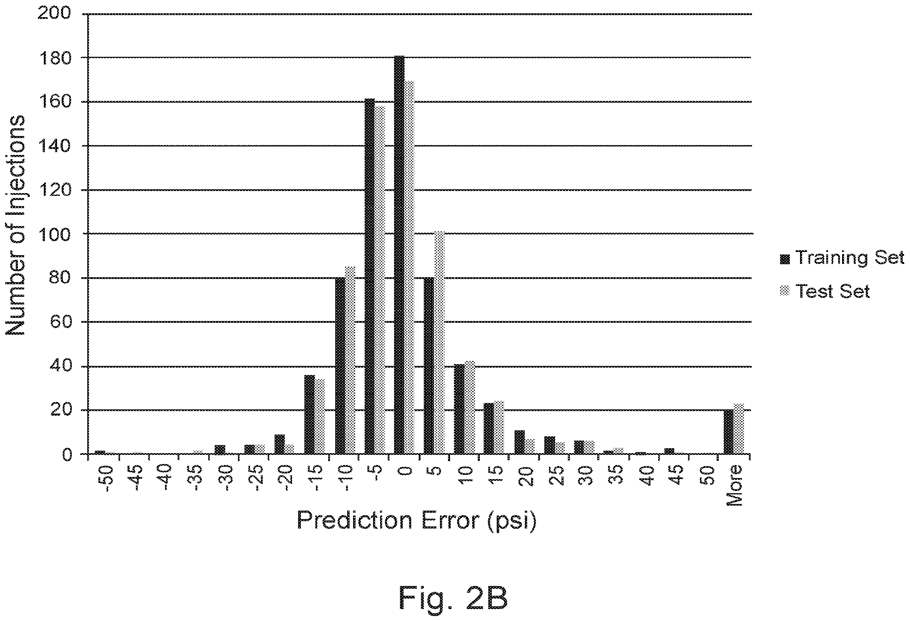

Figure 2B illustrates a histogram of the error in prediction of pressure via a model hereof in a fluid injection apparatus hereof. In the studies of Figure 2B, the maximum or peak pressure range was 0 psi to 359 psi, with an average of approximately 101 psi. The maximum or peak flow rate range was 1.6 ml/s to 8.7 ml/s, with an average of approximately 5.7 ml/s. Catheter type (for example, manufacturer), catheter gauge, contrast type/brand, contrast concentration, peak flow rate, and peak pressure were recorded for 1344 injections from an injection system used in clinical practice. The data was split randomly into two sets - a "training" data set (673 injections) and a "test" data set (673 injections). From the training set, a regression was performed based on the input variables, wherein catheter type was treated as a categorical variable, and contrast brand and concentration were used to estimate viscosity via the lookup table, yielding the following equations:

$$\text{Predicted Pressure if 18 gauge catheter} = -27.3 + 2.5*\text{viscosity} + 17.6*\text{flow} \qquad \text{(Eq. 1)}$$

$$\text{Predicted Pressure if 20 gauge catheter} = -12.7 + 2.5*\text{viscosity} + 17.6*\text{flow} \qquad \text{(Eq. 2)}$$

$$\text{Predicted Pressure if 22 gauge catheter} = -1.6 + 2.5*\text{viscosity} + 17.6*\text{flow} \qquad \text{(Eq. 3)}$$

For both the training data set and the test data set, those equations were used to predict the peak pressure. Residual error was calculated by subtracting the prediction from the actual recorded value, and then plotted in the histogram of Figure 2B. Performance of the prediction on the test data set was comparable to that of the training data set, providing confidence that it could be used prospectively on future injections. As seen in Figure 2B, the above algorithms, although not fully optimized, were quite accurate in predicting peak pressure. Figure 2B and the discussion set forth above demonstrate the manner via which a model including algorithms for various catheter types and gauges can be experimentally developed and/or modified/updated.

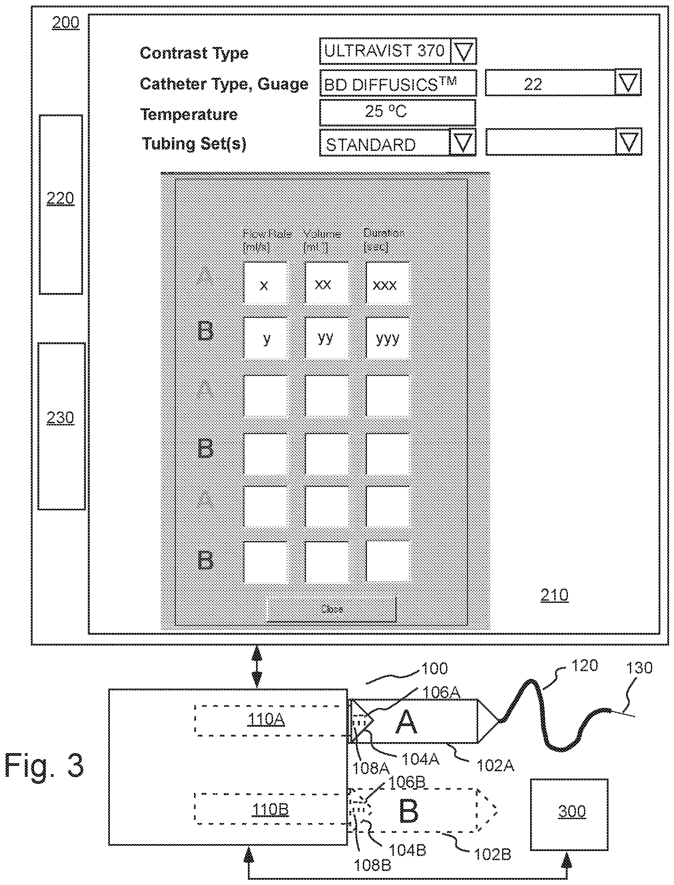

In several representative embodiments hereof, an injection system may be a single syringe injector system or a dual syringe injector system 100 as illustrated in Figure 3 (and as, for example, disclosed in U.S. Patent No. 6,643,537 and U.S. Patent No. 7,553,294). Injector system 100 may, for example, include two fluid delivery sources (sometimes referred to as source "A" and source "B" herein; such as syringes 102A and 102B or other containers) that are operable to introduce a first fluid and/or a second fluid (for example, contrast agent, saline, etc.) to the patient independently (for example, concurrently or simultaneously in the same or different volumetric flow proportion to each other, or sequentially or subsequent to each other (that is, A then B, or B then A)). In the illustrated embodiment, syringes 102A and 102B include plungers 104A and 104B, respectively, which travel axially through syringes 102A and 102B. The devices, systems and methods hereof are also suitable for use in a single source injector system. In the embodiment of Figure 3, source A is operatively connectable to a pressurizing system of injector system 100 including a drive such as a drive member 110A. Likewise, source B is operatively connectable with the pressurizing system via a drive such as a drive member 110B. In the illustrated embodiment, drive member 110A is in operative connection with plunger 104A, while drive member 110B is in operative connection with plunger 104B. Pressurized fluid from, for example, syringe 102A flows to the patient through a flow path including a tubing set 120 and a catheter 130.

The injection system includes a controller or control system 200 in operative connection with drive members 110A and 110B of the pressurizing system and operable to control the operation of drive members 110A and 110B to control injection of fluid A (for example, contrast agent) from source A and injection of fluid B (for example, saline) from source B, respectively. Controller 200 can, for example, include a user interface comprising, for example, a display 210, which may, for example, be a touchscreen display, an audio system, etc. Controller 200 can also include an input system via which data such as injection protocol parameters are input to program an injection protocol. The input system may, for example, include touchscreen display 210, a keypad, a keyboard, a microphone for voice input, a datalink for input from one or more databases, a bar coder reader, an RFID reader and/or other input devices as known in the computer arts. Controller 200 can also include a processor system 220 (for example, including one or more processors such as digital microprocessors as known in the art) in operative connection with a memory system 230, the input system and the user interface.

A model hereof for prediction of pressure may, for example, be stored in computer readable form in non-transitory computer-readable media of memory system 230 and be executable by processor system 220. In general, non-transitory computer-readable media includes all computer-readable media, with the sole exception of a transitory, propagating signal, and includes, for example, CDs, discs, flash drives, RAM, ROM, etc. Injection system 100 may, for example, be in operative connection with an imaging system 300 (for example, a CT imaging system); and one, a plurality or all the components of the injection system and the imaging system 300 may be integrated.

As illustrated in Figure 3, a user may enter flow rates for fluid from source A and source B in each of a plurality of different phases. If the programmed rate in any phase will exceed the achievable rate as determined, for example, via reference to a model incorporating the data (or algorithm(s) as represented by data fit lines or curves) of Figure 2A or Equations 1 through 3 stored in memory system 230, an option may be presented to the user to make a modification. For example, if ULTRA VIST® 370 is to be used with a 22 gauge catheter and a flow rate of 5 ml/s was programmed, the injector may, for example, recommend any one or more of the following actions: changing the 22 gauge catheter to a 20 gauge catheter, warming the contrast, reducing the flow rate to 4.6 ml/s (the maximum achievable with the existing components) or diluting the contrast with a diluent such a saline and increasing the flow rate to compensate for the decreased iodine delivery rate as a result of the dilution.

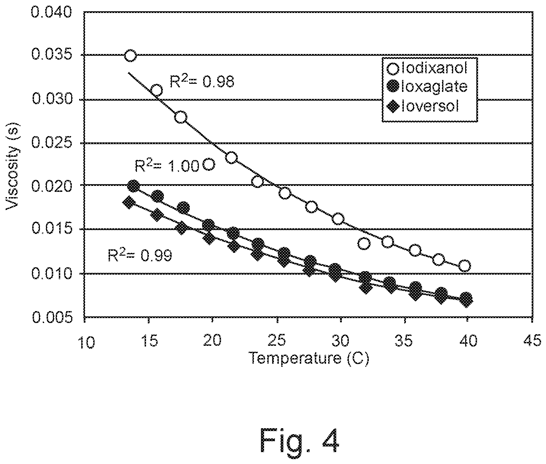

The effect of temperature on the contrast viscosity may be incorporated within the models hereof. For example, Figure 4 illustrates the temperature dependence of viscosity for Iodixanol (a contrast agent, sold under the trade name Visipaque), Ioxaglate (a contrast agent sold under the trade name Hexabrix) and Ioversol (a contrast agent sold under the trade name Optitray). See Brunette, J., et al., "Comparative rheology of low- and iso-osmolarity contrast agents at different temperature", Catheterization and Cardiovascular Interventions, Volume 71, Issue 1, pages 78-83, DOI: 10.1002/ccd.21400 (2007). A temperature dependence model based on studies such as illustrated in Figure 4 may be incorporated in the models hereof.

Injector system 100 may, for example, record (for example, in memory system 230) a peak pressure generated for a given programmed flow rate, contrast, and disposable set characteristics (including catheter characteristics), as well as if the programmed flow rate resulted upon entry into the pressure limiting mode. Pressure may, for example, be measured via one or more pressure sensors 108A and 108B (illustrated schematically in Figure 3) in operative connection with the pressurized fluid. Pressure may, for example, be measured via measuring motor current in drive system(s) 110A and/or 110B, measuring strain on plunger(s) 104A and/or 104B, via a pressure/strain gauge in operative connection with a rubber cover on plunger(s) 104A and/or 104B, or via one or more pressure sensors in fluid connection with the pressurized fluid. Feedback of such data into the models hereof may be used to create and/or improve the models for use at one or more sites.

A model including such data may, for example, be used to predict a peak pressure that will arise in an injection protocol for a future injection procedure. Data from injection procedures may, for example, further optimize a model in a site specific manner or to create a model. For example, if a site consistently uses a tubing setup which is different from the one or more tubing sets used in developing the model, the model may be optimized through data gathered during one or more injection procedures for the tubing setup(s) used in the site. As described above, the manufacturer may, for example, provide injector system 100 inclusive of a model determined, formulated or established with one or more tubing sets of the kind/type that may be recommended for use with injector system 100. However, a site may use an extension set in connection with a tubing set included in the determination of the model or may use a different tubing set. Lower achievable flow rates than the default data in the model will result from use of an extension set. Similarly, if a site uses a tubing set having a larger inner diameter than the tubing set used in developing the model, higher achievable flow rates will result. The models hereof may be developed as described above and/or modified, updated or optimized based upon data from diagnostic and/or other injection procedures. Learning algorithms, as known in the computer arts, may be used to modify, update or optimize the models hereof to accurately predict pressure from planned injection parameters prior to the commencement of injection for many types of tubing set/flow path setups. Moreover, data from multiple sites may be gathered and recorded (for example, by the injector manufacturer) to update/optimize the models hereof. Tubing set configurations may be input by operators and/or sensed via injector sensors to enable standardization of data across different sites. The data feedback loops hereof thus utilize data from previous injections to create, modify, update and/or optimize models hereof (to, for example, optimize the mode in a manner specific to the procedures at a specific clinical site). Moreover, if injections are performed on a given patient prior to the diagnostic injection (for example, a saline patency check or a timing bolus), the pressure data from such injections may be compared to the prediction of the model to further modify/optimize the model.

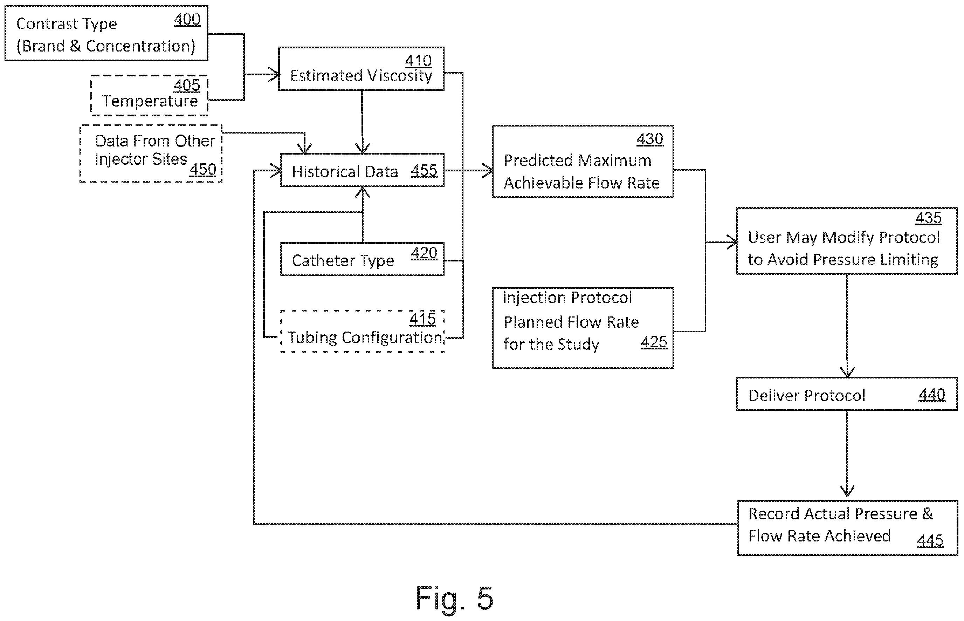

Figure 5 illustrates a flow chart for an embodiment of a methodology hereof. A user may, for example, enter a type of contrast and a concentration to be used in an injection procedure as set forth in item 400 of Figure 5. Such information can also be sensed from information provided from a contrast source (for example, via an RFID tag, a bar code, etc.). In item 405, the user may also enter the temperature of the fluid/contrast to be delivered. Alternatively, temperature may be sensed via a sensor in a fluid heating system which supplies syringe 102A and/or syringe 102B or sensors 106A and 106B (see Figure 3) in operative connection with syringes 102A and 102B, respectively. Sensors 106A and 106B may, for example, be thermocouple and/or infrared temperature sensors positioned within plungers 104A and 104B, respectively. From the identity of the fluid/contrast and the temperature, the system may determine or estimate the fluid viscosity using algorithms/data as set forth in Figure 4 and as set forth in item 410 of Figure 5. Alternatively, the user may directly input the fluid viscosity.

The user may also enter data regarding the configuration of the flow path via the input system of the injector. For example, the user may enter data identifying or characterizing the tubing set(s) connected to sources A and B (for example, syringes 102A and 102B) and the configuration thereof (item 415). In a number of embodiments, a catheter type (item 420) may be characterized by manufacturer and/or by gauge. Different manufacturers may, for example, have catheters that differ in flow characteristics from catheters of the same gauge available from other manufacturers as a result of different inner diameters, different hole configurations, etc. Alternatively, such data may be sensed by one or more sensors (for example, via RFID tags, bar codes, etc.). Likewise, the user may enter data identifying or characterizing the catheter being used. Once again, such data may be sensed by one or more sensors.

The user then may enter the parameters of the injection protocol including the planned or targeted flow rate (item 425). As used herein with respect to an injection procedure, the term "protocol" refers to a group of parameters such as flow rate, volume injected, duration, etc. that define the amount of fluid(s) to be delivered to a patient during an injection procedure. Such parameters can change over the course of the injection procedure. As used herein, the term "phase" refers generally to a group of parameters that define the amount of fluid(s) to be delivered to a patient during a period of time (or phase duration) that can be less than the total duration of the injection procedure. Thus, the parameters of a phase provide a description of the injection over a time instance corresponding to the time duration of the phase. An injection protocol for a particular injection procedure can, for example, be described as uniphasic (a single phase), biphasic (two phases) or multiphasic (two or more phases, but typically more than two phases). Multiphasic injections also include injections in which the parameters can change continuously over at least a portion of the injection procedure. A number of the parameters of an injection protocol may, for example, be determined by a parameter generation system or model stored in memory system 230 and executable by processor system 220 as, for example, described in International Patent Application Publication No. WO 2008/085421 (e.g., based at least in part on one or more patient parameters such as weight, body mass index, cardiac output, blood volume, etc.).

The model(s) hereof may, for example, determine a predicted maximum achievable flow rate as set forth in item 430 (based upon a predetermined pressure limit or pressure threshold value). The model may further determine if a planned flow rate for any phase of a planned injection protocol exceeds the maximum achievable flow rate (based upon predetermined pressure threshold value) for the contrast viscosity and flow path configuration. Alternatively, the models hereof may determine a predicted maximum pressure for the planned injection protocol, contrast viscosity and flow path configuration in each phase and determine if, for any phase, the predicted maximum pressure exceeds the predetermined pressure threshold value. Determining if a planned flow rate exceeds a maximum achievable flow rate (based upon a predetermined pressure threshold value) and if a predicted maximum pressure exceeds a predetermined pressure threshold are essentially equivalent analyses and are referred to collectively herein as determining if a predicted maximum pressure exceeds a predetermined pressure threshold value.

If the flow rate in any phase exceeds the maximum achievable flow rate, the user may be provided the opportunity to change parameters of the injection protocol to avoid pressure limiting (item 435). If that is the case, the clinician has several options: use a lower viscosity contrast agent, insert a larger catheter, warm the contrast, dilute the contrast with a diluent or reduce the programmed flow rate of the protocol and adjust the scan timing. If the parameters of the injection protocol are changed, the model may repeat the process of determining if the flow rate in any phase exceeds the maximum achievable flow rate. Once the flow rate in each phase does not exceed the maximum achievable flow rate, the injection procedure may proceed with the indicated injection protocol (item 440). The injection procedure can also be continued while allowing pressure limiting to occur. The model may also automatically effect a change (that is, a parameter change) to update the initial injection protocol to create an updated injection protocol so that the expected pressure level will be determined via the model to be less than or equal to the pressure threshold value. The updated injection protocol may then be used to inject the fluid. Once the injection protocol is carried out and the fluid delivered to the patient, the actual pressure and flow rate achieved may be recorded as set forth in item 445 and used by the model to modify, update and/or optimize the model. Data from other injector apparatuses or sites (item 450) may be combined with data from previous injection procedures on the injector apparatus including the model as historical data (item 455) via which the model may be modified or optimized.

As described above, the models of the present invention are predictive of pressure in newly installed flow paths without previous characterization of the installed flow path based upon experimental results with other flow paths/tubing sets of the same or similar design (that is, of like kind). Moreover, the models are adaptive to changes in flow paths as well as to installation of flow paths of completely different design. The models of the present invention are more accurate than models which attempt to calculate pressure in flow paths by solving equations derived from theoretical fluid dynamics and are more readily updated or optimized using data from actual injection procedures.

**Claims**

1. A fluid injection apparatus comprising:

   at least one pressurizing system;
   at least a first fluid path operably connectible to the at least one pressurizing system to transport fluid pressurized by the pressurizing system, the at least a first fluid path comprising a tubing set and a catheter;
   a control system operably associated with the at least one pressurizing system and comprising an input system, a user interface system, a processor system, and a memory system, the input system providing for input of an initial injection protocol of an injection procedure according to which at least one fluid is intended to be injected into a patient, and
   at least one model stored in the memory system and executable by the processor system to predict, before carrying out the initial injection protocol, if a pressure threshold value would be reached in the at least a first fluid path if the initial injection protocol were carried out, the at least one model determining if the pressure threshold value would be reached on the basis of a flow rate of the at least one fluid, at least one catheter characteristic, and a viscosity of the at least one fluid, the at least one model being determined experimentally for a plurality of fluids of different viscosities, for a plurality of catheters of different catheter characteristics, and for at least one tubing set of like kind to the tubing set of the first fluid path,

   upon determining via the at least one model that the pressure threshold value would not be reached, the processor system enabling the initial injection protocol to be carried out by the fluid injection apparatus, and upon determining via the at least one model that the pressure threshold value will be reached, the processor system either (I) alerting an operator via the user interface system, whereupon the operator is enabled to choose between (a) permitting the initial injection protocol to be carried out by the fluid injection apparatus as programmed and (b) effecting a change to update the initial injection protocol for input into the at least one model or (II) automatically effecting a change to update the initial injection protocol to create an updated injection protocol so that the expected pressure level will be determined via the at least one model to be less than or equal to the pressure threshold value thereby enabling the updated injection protocol to be used to introduce the at least one fluid into the fluid path.

2. The fluid injection apparatus of claim 1 wherein effecting a change to update the initial injection protocol for input into the at least one model comprises at least one of (a) changing the flow rate of the fluid, (b) changing the at least one catheter characteristic, and (c) changing the viscosity of the at least one fluid.

3. The fluid injection apparatus of claim 2 wherein the at least one model is adapted to receive data from at least one previous injection procedure with the fluid injection apparatus to update the at least one model on the basis of the data from the at least one previous injection procedure with the fluid injection apparatus.

4. The fluid injection apparatus of claim 3 wherein the at least one model is adapted to receive data from at least one previous injection procedure with another fluid injection apparatus to update the at least one model on the basis of the data from the at least one previous injection procedure with the another fluid injection apparatus.

5. The fluid injection apparatus of claim 2 wherein the pressure threshold value is set by one of (i) the operator of the fluid injection apparatus and (ii) a manufacturer of the fluid injection apparatus.

6. The fluid injection apparatus of claim 2 wherein, if it is determined via the at least one model that the pressure threshold value would be reached if the initial injection protocol were carried out, the effecting of a change to update the initial injection protocol as chosen by the operator includes at least one of (i) a flow rate of the at least one fluid, (ii) a concentration of the at least one fluid, (iii) a ratio of one fluid of the at least one fluid to another fluid of the at least one fluid and (iv) a temperature of the at least one fluid.

7. The fluid injection apparatus of claim 2 wherein the at least one model is determined experimentally for a plurality of fluids of different viscosities and for a plurality of catheters of different gauges used in connection with a plurality of tubing sets, wherein at least one of the plurality of tubing sets is of a like kind to the tubing set.

8. A method of predicting peak pressure within a fluid path comprising a tubing set and a catheter into which at least one fluid is introduced under pressure by a pressurizing system of a fluid injection apparatus, the method comprising:

(a) inputting an initial injection protocol into the fluid injection apparatus according to which the at least one fluid is intended to be introduced into the fluid path;
(b) predicting an expected peak pressure level that would result in the fluid path if the initial injection protocol were to be used as intended to introduce the at least one fluid into the fluid path, the expected peak pressure level being determined before commencement of the initial injection protocol according to at least one model on the basis of a flow rate of the at least one fluid, at least one catheter characteristic, and a viscosity of the at least one fluid, the at least one model being determined experimentally for a plurality of fluids of different viscosities, for a plurality of catheters of different catheter characteristics, and for at least one tubing set of like kind to the tubing set of the fluid path.

9. The method of claim 8, further comprising

(c) if the expected pressure level is less than or equal to a pressure threshold value, enabling the initial injection protocol to be used as intended to introduce the at least one fluid into the fluid path; and
(d) if the expected pressure level exceeds the pressure threshold value, performing one of:

(I) alerting an operator of the method thereof whereupon the operator is enabled to choose between one of (i) permitting the initial injection protocol to be used as intended to introduce the at least one fluid into the fluid path and (ii) effecting a change to update the initial injection protocol for input into the at least one model; and
(II) automatically effecting a change to update the initial injection protocol to create an updated injection protocol so that the expected pressure level will be determined via the at least one model to be less than or equal to the pressure threshold value thereby enabling the updated injection protocol to be used to introduce the at least one fluid into the fluid path.

10. The method of claim 9 wherein effecting a change to update the initial injection protocol for input into the at least one model comprises changing the flow rate of the at least one fluid, changing the at least one catheter characteristic, or changing the viscosity of the at least one fluid.

11. The method of claim 10 further comprising inputting data from at least one previous injection procedure with the fluid injection apparatus into the at least one model and updating the at least one model on the basis of the data from the at least one previous injection procedure with the fluid injection apparatus.

12. The method of claim 10 further comprising inputting data from at least one previous injection procedure with another fluid injection apparatus into the at least one model and updating the at least one model on the basis of the data from the at least one previous injection procedure with the another fluid injection apparatus.

13. The method of claim 10 wherein the pressure threshold value is set by one of (i) the operator of the fluid injection apparatus and (ii) a manufacturer of the fluid injection apparatus.

14. The method of claim 10 wherein, if it is determined via the at least one model that the pressure threshold value would be reached if the initial injection protocol were carried out, the effecting of a change to update the initial injection protocol as chosen by the operator includes at least one of (i) a flow rate of the at least one fluid, (ii) a concentration of the at least one fluid, a (iii) a ratio of one fluid of the at least one fluid to another fluid of the at least one fluid and (iv) a temperature of the at least one fluid.

15. The method of claim 10 wherein the at least one model is determined experimentally for a plurality of fluids of different viscosities and for a plurality of catheters of different gauges used in connection with a plurality of tubing sets, wherein at least one of the plurality of tubing sets is of a like kind to the tubing set.

**Patentansprüche**

1. Flüssigkeitsinjektionsvorrichtung, umfassend:

mindestens ein Druckbeaufschlagungssystem;
mindestens einen ersten Flüssigkeitsweg, der funktionsfähig mit dem mindestens einen Druckbeaufschlagungssystem verbindbar ist, um Flüssigkeit zu transportieren, die vom Druckbeaufschlagungssystem mit Druck beaufschlagt wird, wobei der mindestens eine Flüssigkeitsweg ein Schlauchset und einen Katheter umfasst;
ein Steuersystem, das mit dem mindestens einen Druckbeaufschlagungssystem in funktionsfähiger Verbindung steht und ein Eingabesystem, ein Benutzerschnittstellensystem, ein Prozessorsystem und ein Speichersystem umfasst, wobei das Eingabesystem die Eingabe eines anfänglichen Injektionsprotokolls eines Injektionsverfahrens, gemäß dem mindestens eine Flüssigkeit in einen Patienten injiziert werden soll, ermöglicht, und mindestens ein Modell, das im Speichersystem gespeichert ist und vom Prozessorsystem ausführbar ist, um vor der Durchführung des anfänglichen Injektionsprotokolls vorherzusagen, ob ein Druckschwellenwert in dem mindestens einen ersten Flüssigkeitsweg bei Durchführung des anfänglichen Injektionsprotokolls erreicht werden würde, wobei das mindestens eine Modell feststellt, ob der Druckschwellenwert auf Basis einer Strömungsrate der mindestens einen Flüssigkeit, mindestens einer Kathetereigenschaft und einer Viskosität der mindestens einen Flüssigkeit erreicht werden würde, wobei das mindestens eine Modell experimentell für eine Vielzahl von Flüssigkeiten mit unterschiedlichen Viskositäten, für eine Vielzahl von Kathetern mit unterschiedlichen Kathetereigenschaften und für mindestens ein Schlauchset ähnlicher Art wie das Schlauchset des ersten Flüssigkeitswegs bestimmt wird,

wobei, wenn festgestellt wird, über das mindestens eine Modell, dass der Druckschwellenwert nicht erreicht werden würde, das Prozessorsystem die Durchführung des anfänglichen Injektionsprotokolls durch die Flüssigkeitsinjektionsvorrichtung aktiviert, und wenn festgestellt wird, über das mindestens eine Modell, dass der Druckschwellenwert erreicht wird, das Prozessorsystem entweder (I) eine Bedienungsperson über das Benutzerschnittstellensystem benachrichtigt, woraufhin die Bedienungsperson die Möglichkeit hat zu wählen zwischen (a) Zulassen der Durchführung des anfänglichen Injektionsprotokolls durch die Flüssigkeitsinjektionsvorrichtung wie programmiert und (b) Bewirken einer Änderung zur Aktualisierung des anfänglichen Injektionsprotokolls zur Eingabe in das mindestens eine Modell, oder (II) automatisch eine Änderung zur Aktualisierung des anfänglichen Injektionsprotokolls bewirkt, um ein aktualisiertes Injektionsprotokoll zu erzeugen, so dass das erwartete Druckniveau über das mindestens eine Modell als kleiner als oder gleich dem Druckschwellenwert festgestellt wird, wodurch die Verwendung des aktualisierten Injektionsprotokolls zur Einleitung der mindestens einen Flüssigkeit in den Flüssigkeitsweg ermöglicht wird.

2. Flüssigkeitsinjektionsvorrichtung nach Anspruch 1, wobei das Bewirken einer Änderung zur Aktualisierung des anfänglichen Injektionsprotokolls zur Eingabe in das mindestens eine Modell mindestens eines von (a) Ändern der Strömungsrate der Flüssigkeit, (b) Ändern der mindestens einen Kathetereigenschaft und (c) Ändern der Viskosität der mindestens einen Flüssigkeit umfasst.

3. Flüssigkeitsinjektionsvorrichtung nach Anspruch 2, wobei das mindestens eine Modell zum Empfangen von Daten von mindestens einem vorherigen Injektionsverfahren mit der Flüssigkeitsinjektionsvorrichtung angepasst ist, um das mindestens eine Modell auf Basis der Daten von dem mindestens einen vorherigen Injektionsverfahren mit der Flüssigkeitsinjektionsvorrichtung zu aktualisieren.

4. Flüssigkeitsinjektionsvorrichtung nach Anspruch 3, wobei das mindestens eine Modell zum Empfangen von Daten von mindestens einem vorherigen Injektionsverfahren mit einer anderen Flüssigkeitsinjektionsvorrichtung angepasst ist, um das mindestens eine Modell auf Basis der Daten von dem mindestens einen vorherigen Injektionsverfahren mit der anderen Flüssigkeitsinjektionsvorrichtung zu aktualisieren.

5. Flüssigkeitsinjektionsvorrichtung nach Anspruch 2, wobei der Druckschwellenwert durch (i) die Bedienungsperson der Flüssigkeitsinjektionsvorrichtung oder (ii) einen Hersteller der Flüssigkeitsinjektionsvorrichtung eingestellt wird.

6. Flüssigkeitsinjektionsvorrichtung nach Anspruch 2, wobei, wenn über das mindestens eine Modell festgestellt wird, dass der Druckschwellenwert bei Durchführung des anfänglichen Injektionsprotokolls erreicht werden würde, das Bewirken einer Änderung zur Aktualisierung des anfänglichen Injektionsprotokolls wie von der Bedienungsperson gewählt mindestens eines von (i) einer Strömungsrate der mindestens einen Flüssigkeit, (ii) einer Konzentration der mindestens einen Flüssigkeit, (iii) einem Verhältnis einer Flüssigkeit der mindestens einen Flüssigkeit zu einer anderen Flüssigkeit der mindestens einen Flüssigkeit und (iv) einer Temperatur der mindestens einen Flüssigkeit beinhaltet.

7. Flüssigkeitsinjektionsvorrichtung nach Anspruch 2, wobei das mindestens eine Modell experimentell für eine Vielzahl von Flüssigkeiten mit unterschiedlichen Viskositäten und für eine Vielzahl von Kathetern mit unterschiedlichen Gauge-Werten, die in Verbindung mit einer Vielzahl von Schlauchsets verwendet werden, bestimmt wird, wobei mindestens eines der Vielzahl von Schlauchsets von ähnlicher Art wie das Schlauchset ist.

8. Verfahren zur Vorhersage eines Spitzendrucks in einem Flüssigkeitsweg, der ein Schlauchset und einen Katheter umfasst, in den mindestens eine Flüssigkeit unter Druck von einem Druckbeaufschlagungssystem einer Flüssigkeitsinjektionsvorrichtung eingeleitet wird, wobei das Verfahren das Folgende umfasst:

(a) Eingabe eines anfänglichen Injektionsprotokolls in die Flüssigkeitsinjektionsvorrichtung, gemäß dem die mindestens eine Flüssigkeit in den Flüssigkeitsweg eingeleitet werden soll;

(b) Vorhersage eines erwarteten Spitzendruckniveaus, das im Flüssigkeitsweg resultieren würde, wenn das anfängliche Injektionsprotokoll wie beabsichtigt zur Einleitung der mindestens einen Flüssigkeit in den Flüssigkeitsweg verwendet werden würde, wobei das erwartete Spitzendruckniveau vor Beginn des anfänglichen Injektionsprotokolls gemäß mindestens einem Modell auf Basis einer Strömungsrate der mindestens einen Flüssigkeit, mindestens einer Kathetereigenschaft und einer Viskosität der mindestens einen Flüssigkeit bestimmt wird, wobei das mindestens eine Modell experimentell für eine Vielzahl von Flüssigkeiten mit unterschiedlichen Viskositäten, für eine Vielzahl von Kathetern mit unterschiedlichen Kathetereigenschaften und für mindestens ein Schlauchset ähnlicher Art wie das Schlauchset des Flüssigkeitswegs bestimmt wird.

9. Verfahren nach Anspruch 8, ferner umfassend:

(c) wenn das erwartete Druckniveau kleiner als oder gleich einem Druckschwellenwert ist, Erlauben der Verwendung des anfänglichen Injektionsprotokolls wie beabsichtigt zur Einleitung der mindestens einen Flüssigkeit in den Flüssigkeitsweg; und

(d) wenn das erwartete Druckniveau den Druckschwellenwert überschreitet, Durchführen eines von:

(I) Benachrichtigen einer Bedienungsperson des Verfahrens darüber, woraufhin die Bedienungsperson die Möglichkeit hat zu wählen zwischen (i) Zulassen der Verwendung des anfänglichen Injektionsprotokolls wie beabsichtigt zur Einleitung der mindestens einen Flüssigkeit in den Flüssigkeitsweg und (ii) Bewirken einer Änderung zur Aktualisierung des anfänglichen Injektionsprotokolls zur Eingabe in das mindestens eine Modell; und

(II) automatisches Bewirken einer Änderung zur Aktualisierung des anfänglichen Injektionsprotokolls, um ein aktualisiertes Injektionsprotokoll zu erzeugen, so dass das erwartete Druckniveau über das mindestens eine Modell als kleiner als oder gleich dem Druckschwellenwert festgestellt wird, wodurch die Verwendung des aktualisierten Injektionsprotokolls zur Einleitung der mindestens einen Flüssigkeit in den Flüssigkeitsweg ermöglicht wird.

10. Verfahren nach Anspruch 9, wobei das Bewirken einer Änderung zur Aktualisierung des anfänglichen Injektionsprotokolls zur Eingabe in das mindestens eine Modell Ändern der Strömungsrate der mindestens einen Flüssigkeit, Ändern der mindestens einen Kathetereigenschaft oder Ändern der Viskosität der mindestens einen Flüssigkeit umfasst.

11. Verfahren nach Anspruch 10, ferner umfassend die Eingabe von Daten von mindestens einem vorherigen Injektionsverfahren mit der Flüssigkeitsinjektionsvorrichtung in das mindestens eine Modell und Aktualisierung des mindestens einen Modells auf Basis der Daten von dem mindestens einen vorherigen Injektionsverfahren mit der Flüssigkeitsinjektionsvorrichtung.

**12.** Verfahren nach Anspruch 10, ferner umfassend die Eingabe von Daten von mindestens einem vorherigen Injektionsverfahren mit einer anderen Flüssigkeitsinjektionsvorrichtung in das mindestens eine Modell und Aktualisierung des mindestens einen Modells auf Basis der Daten von dem mindestens einen vorherigen Injektionsverfahren mit der anderen Flüssigkeitsinjektionsvorrichtung.

**13.** Verfahren nach Anspruch 10, wobei der Druckschwellenwert durch (i) die Bedienungsperson der Flüssigkeitsinjektionsvorrichtung oder (ii) einen Hersteller der Flüssigkeitsinjektionsvorrichtung eingestellt wird.

**14.** Verfahren nach Anspruch 10, wobei, wenn über das mindestens eine Modell festgestellt wird, dass der Druckschwellenwert bei Durchführung des anfänglichen Injektionsprotokolls erreicht werden würde, das Bewirken einer Änderung zur Aktualisierung des anfänglichen Injektionsprotokolls wie von der Bedienungsperson gewählt mindestens eines von (i) einer Strömungsrate der mindestens einen Flüssigkeit, (ii) einer Konzentration der mindestens einen Flüssigkeit, (iii) einem Verhältnis einer Flüssigkeit der mindestens einen Flüssigkeit zu einer anderen Flüssigkeit der mindestens einen Flüssigkeit und (iv) einer Temperatur der mindestens einen Flüssigkeit beinhaltet.

**15.** Verfahren nach Anspruch 10, wobei das mindestens eine Modell experimentell für eine Vielzahl von Flüssigkeiten mit unterschiedlichen Viskositäten und für eine Vielzahl von Kathetern mit unterschiedlichen Gauge-Werten, die in Verbindung mit einer Vielzahl von Schlauchsets verwendet werden, bestimmt wird, wobei mindestens eines der Vielzahl von Schlauchsets von ähnlicher Art wie das Schlauchset ist.


**Revendications**

**1.** Appareil d'injection de fluide comprenant :

au moins un système de mise sous pression ;
au moins un premier trajet de fluide pouvant être raccordé fonctionnellement à l'au moins un système de mise sous pression pour transporter un fluide mis sous pression par le système de mise sous pression, l'au moins un premier trajet de fluide comprenant un ensemble de tubes et un cathéter ;
un système de commande associé fonctionnellement à l'au moins un système de mise sous pression et comprenant un système d'entrée, un système d'interface utilisateur, un système de processeur et un système de mémoire, le système d'entrée permettant une entrée d'un protocole d'injection initial d'une procédure d'injection selon lequel au moins un fluide est destiné à être injecté dans un patient, et
au moins un modèle mémorisé dans le système de mémoire et pouvant être exécuté par le système de processeur pour prédire, avant de réaliser le protocole d'injection initial, si une valeur de seuil de pression serait atteinte dans l'au moins un premier trajet de fluide si le protocole d'injection initial était effectué, l'au moins un modèle déterminant si la valeur de seuil de pression serait atteinte sur la base d'un débit de l'au moins un fluide, d'au moins une caractéristique de cathéter et d'une viscosité de l'au moins un fluide, l'au moins un modèle étant déterminé expérimentalement pour une pluralité de fluides ayant des viscosités différentes, pour une pluralité de cathéters ayant des caractéristiques de cathéter différentes, et pour au moins un ensemble de tubes d'un type identique à celui de l'ensemble de tubes du premier trajet de fluide, lors de la détermination par l'intermédiaire de l'au moins un modèle que la valeur de seuil de pression ne serait pas atteinte, le système de processeur permettant que le protocole d'injection initial soit réalisé par l'appareil d'injection de fluide, et lors de la détermination par l'intermédiaire de l'au moins un modèle que la valeur de seuil de pression sera atteinte, le système de processeur soit (I) alertant un opérateur par l'intermédiaire du système d'interface utilisateur, après quoi l'opérateur peut choisir entre (a) permettre que le protocole d'injection initial soit effectué par l'appareil d'injection de fluide comme programmé et (b) effectuer un changement pour mettre à jour le protocole d'injection initial pour une entrée dans l'au moins un modèle, soit (II) effectuant automatiquement un changement pour mettre à jour le protocole d'injection initial dans le but de créer un protocole d'injection mis à jour de sorte que le niveau de pression attendu soit déterminé par l'intermédiaire de l'au moins un modèle comme étant inférieur ou égal à la valeur de seuil de pression, ce qui permet que le protocole d'injection mis à jour soit utilisé pour introduire l'au moins un fluide dans le trajet de fluide.

**2.** Appareil d'injection de fluide selon la revendication 1, dans lequel le fait d'effectuer un changement pour mettre à jour le protocole d'injection initial pour une entrée dans l'au moins un modèle consiste à (a) changer le débit du fluide et/ou (b) changer l'au moins une caractéristique de cathéter et/ou (c) changer la viscosité de l'au moins un fluide.

**3.** Appareil d'injection de fluide selon la revendication 2, dans lequel l'au moins un modèle est conçu pour recevoir des données en provenance de l'au moins une procédure d'injection précédente avec l'appareil d'injection de fluide pour mettre à jour l'au moins un modèle sur la base des données en provenance de l'au moins une procédure d'injection précédente avec l'appareil d'injection de fluide.

4. Appareil d'injection de fluide selon la revendication 3, dans lequel l'au moins un modèle est conçu pour recevoir des données en provenance de l'au moins une procédure d'injection précédente avec un autre appareil d'injection de fluide pour mettre à jour l'au moins un modèle sur la base des données en provenance de l'au moins une procédure d'injection précédente avec l'autre appareil d'injection de fluide.

5. Appareil d'injection de fluide selon la revendication 2, dans lequel la valeur de seuil de pression est définie par (i) l'opérateur de l'appareil d'injection de fluide ou (ii) un fabricant de l'appareil d'injection de fluide.

6. Appareil d'injection de fluide selon la revendication 2, dans lequel, s'il est déterminé par l'intermédiaire de l'au moins un modèle que la valeur de seuil de pression serait atteinte si le protocole d'injection initial était effectué, le fait d'effectuer un changement pour mettre à jour le protocole d'injection initial selon le choix de l'opérateur comprend au moins un parmi (i) un débit de l'au moins un fluide, (ii) une concentration de l'au moins un fluide, (iii) un rapport d'un fluide de l'au moins un fluide à un autre fluide de l'au moins un fluide et (iv) une température de l'au moins un fluide.

7. Appareil d'injection de fluide selon la revendication 2, dans lequel l'au moins un modèle est déterminé expérimentalement pour une pluralité de fluides ayant des viscosités différentes et pour une pluralité de cathéters ayant des calibres différents utilisés en liaison avec une pluralité d'ensembles de tubes, dans lequel au moins un ensemble de la pluralité d'ensembles de tubes est d'un type identique à celui de l'ensemble de tubes.

8. Procédé de prédiction d'un pic de pression à l'intérieur d'un trajet de fluide comprenant un ensemble de tubes et un cathéter dans lequel au moins un fluide est introduit sous pression par un système de mise sous pression d'un appareil d'injection de fluide, le procédé consistant à :

(a) entrer un protocole d'injection initial dans l'appareil d'injection de fluide selon lequel l'au moins un fluide est destiné à être introduit dans le trajet de fluide ;
(b) prédire un niveau de pic de pression attendu qui serait obtenu dans le trajet de fluide si le protocole d'injection initial devait être utilisé comme prévu pour introduire l'au moins un fluide dans le trajet de fluide, le niveau de pic de pression attendu étant déterminé avant le début du protocole d'injection initial selon au moins un modèle sur la base d'un débit de l'au moins un fluide, d'au moins une caractéristique de cathéter, et d'une viscosité de l'au moins un fluide, l'au moins un modèle étant déterminé expérimentalement pour une pluralité de fluides ayant des viscosités différentes, pour une pluralité de cathéters ayant des caractéristiques de cathéter différentes, et pour au moins un ensemble de tubes d'un type identique à celui de l'ensemble de tubes du trajet de fluide.

9. Procédé selon la revendication 8, consistant en outre à

(c) si le niveau de pression attendu est inférieur ou égal à une valeur de seuil de pression, permettre que le protocole d'injection initial soit utilisé comme prévu pour introduire l'au moins un fluide dans le trajet de fluide ; et
(d) si le niveau de pression attendu dépasse la valeur de seuil de pression, réaliser un parmi ce qui suit :

(I) alerter un opérateur du procédé associé, après quoi l'opérateur peut choisir entre (i) permettre que le protocole d'injection initial soit utilisé comme prévu pour introduire l'au moins un fluide dans le trajet de fluide et (ii) effectuer un changement pour mettre à jour le protocole d'injection initial pour une entrée dans l'au moins un modèle ; et
(II) automatiquement effectuer un changement pour mettre à jour le protocole d'injection initial dans le but de créer un protocole d'injection mis à jour de sorte que le niveau de pression attendu soit déterminé par l'intermédiaire de l'au moins un modèle comme étant inférieur ou égal à la valeur de seuil de pression, ce qui permet ainsi que le protocole d'injection mis à jour soit utilisé pour introduire l'au moins un fluide dans le trajet de fluide.

10. Procédé selon la revendication 9, dans lequel le fait d'effectuer un changement pour mettre à jour le protocole d'injection initial pour une entrée dans l'au moins un modèle consiste à changer le débit de l'au moins un fluide, changer l'au moins une caractéristique de cathéter ou changer la viscosité de l'au moins un fluide.

**11.** Procédé selon la revendication 10, consistant en outre à entrer des données provenant d'au moins une procédure d'injection précédente avec l'appareil d'injection de fluide dans l'au moins un modèle et à mettre à jour l'au moins un modèle sur la base des données en provenance de l'au moins une procédure d'injection précédente avec l'appareil d'injection de fluide.

**12.** Procédé selon la revendication 10, consistant en outre à saisir des données provenant d'au moins une procédure d'injection précédente avec un autre appareil d'injection de fluide dans l'au moins un modèle et à mettre à jour l'au moins un modèle sur la base des données en provenance de l'au moins une procédure d'injection précédente avec l'autre appareil d'injection de fluide.

**13.** Procédé selon la revendication 10, dans lequel la valeur de seuil de pression est définie par (i) l'opérateur de l'appareil d'injection de fluide ou (ii) un fabricant de l'appareil d'injection de fluide.

**14.** Procédé selon la revendication 10 dans lequel, s'il est déterminé par l'intermédiaire de l'au moins un modèle que la valeur de seuil de pression serait atteinte si le protocole d'injection initial était effectué, le fait d'effectuer un changement pour mettre à jour le protocole d'injection initial selon le choix de l'opérateur comprend au moins un parmi (i) un débit de l'au moins un fluide, (ii) une concentration de l'au moins un fluide, (iii) un rapport d'un fluide de l'au moins un fluide à un autre fluide de l'au moins un fluide et (iv) une température de l'au moins un fluide.

**15.** Procédé selon la revendication 10, dans lequel l'au moins un modèle est déterminé expérimentalement pour une pluralité de fluides ayant des viscosités différentes et pour une pluralité de cathéters ayant des calibres différents utilisés en liaison avec une pluralité d'ensembles de tubes, dans lequel au moins un ensemble de la pluralité d'ensembles de tubes est d'un type identique à celui de l'ensemble de tubes.

**Fig. 1**

**Fig. 2A**

Fig. 2B

Fig. 3

Fig. 4

Fig. 5

EP 3 426 154 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2990073 A1
- WO 2013043868 A
- US 2006079765 A1

- US 6643537 B
- US 7553294 B
- WO 2008085421 A

**Non-patent literature cited in the description**

- **BRUNETTE, J. et al.** Comparative rheology of low- and iso-osmolarity contrast agents at different temperature. *Catheterization and Cardiovascular Interventions,* 2007, vol. 71 (1), 78-83